# EUROPEAN PATENT APPLICATION

(11) **EP 1 270 003 A1**
(43) Date of publication of application: **02.01.2003**
(21) Application number: 02254210.4
(22) Date of filing: 17.06.2002
(51) Int. Cl.: A61K 31/198, A61P 43/00

(54) **Use of amino acids such as arginine or citrulline for improving the hatchability of eggs**

(30) Priority: 22.06.2001 US 886086
(71) Applicant: Nutri-Quest, Inc., Chesterfield, MO 63017 (US)
(72) Inventor: Furukawa, Satoru, c/o Nutri-Quest Inc., Chesterfield, MO 63017 (US); Kidd, Michael T., Starksville, MS 39579 (US)
(74) Representative: Harding, Charles Thomas

(57) **Abstract**

The present invention relates to a method of improving the hatchability of eggs and a composition used for the method. The present invention provides the method comprising administrating an amino acid solution into hatching eggs and a composition for improving the hatchability of eggs which contains an amino acid as an effective ingredient.

## Description

### BACKGROUND OF THE INVENTION

### Field of the Invention

The present invention relates to a method of improving the hatchability of eggs by administrating an amino acid- containing solution to hatching eggs as well as a composition containing an amino acid for improving the hatchability of eggs.

### Description of the Related Art

Improvement in the hatchability of hatching eggs is an important object in poultry raising since this leads to the improvement in productivity of chickens. The hatchability in poultry yards has been improved by controlling temperature and humidity so far, however, a method of improving the hatchability by administrating nutrient substances to eggs has not yet been known.

Embrex Co. has developed a method of efficiently injecting vaccines to hatching eggs with an aim of lowering the mortality of chickens, and the method has been generally used mainly in the United States. Administration of vaccines to hatching eggs intends to form antibodies by the vaccines before hatching thereby providing protective immunity after hatching, for example, to Infectious Bursal Disease Virus, but it has no effect for improving the hatchability.

It has been known that the body weight of chickens upon hatching is increased by the administration of a mixed solution of amino acids to hatching eggs (Poultry Science, 78, 1493 - 1498, 1999; British Poultry Science, 23, 171 - 174, 1982).

### SUMMARY OF THE INVENTION

The present invention relates to a method of improving the hatchability of eggs by administrating an amino acid- containing solution to hatching eggs as well as a composition containing an amino acid for improving the hatchability of eggs.

The present inventors have made earnest study to achieve the foregoing object and accomplished the present invention. That is, the present invention relates to the followings (1) - (13).
(1) Amethod of improving the hatchability of eggs, which comprises administrating an amino acid solution to hatching eggs.
(2) A method as defined in (1) above, wherein the amino acid is a basic amino acid.
(3) A method as defined in (2) above, wherein the basic amino acid is arginine or citrulline.
(4) A method as defined in (1) or (2) above, wherein the amino acid solution is administrated together with a vaccine.
(5) A method as defined in any one of (1) to (4) above, wherein the concentration of the amino acid in the amino acid solution is 0.01% (w/v) to 50% (w/v).
(6) A method as defined in any one of (1) to (5) above, wherein the amount of amino acid to be administrated is 0.01 mg to 1 g per one administration.
(7) A composition for improving the hatchability of eggs which contains an amino acid as an effective ingredient.
(8) A composition as defined in (7) above, wherein the amino acid is a basic amino acid.
(9) A composition as defined in (7) or (8) above, wherein the basic amino acid is arginine or citrulline.
(10) A composition as defined in any one of (7) to (9) above, wherein the composition contains a vaccine.
(11) A composition as defined in any one of (7) to (10) above, wherein the concentration of the amino acid is 0.01% (w/v) to 50% (w/v).
(12) A composition as defined in any one of (7) to (11) above, wherein the composition is in the form of an aqueous solution.
(13) A composition as defined in any one of (7) to (11) above, wherein the composition is in the form of a freeze-dried product.
(14) A composition as defined in (12) above, wherein the aqueous solution is obtained by dissolving the composition as defined in (13) above in water.

### DETAILED DESCRIPTION OF THE INVENTION

### 1. Composition for improving the hatchability of eggs

The composition used for improving the hatchability of eggs according to the present invention includes a solution containing amino acids shown below as an effective ingredient or a freeze-dried product obtained by freeze drying the solution.

As the amino acid, any amino acids may be used, and basic amino acids are used preferably. As the basic amino acid, arginine, citrulline, and the like are used alone or in combination. Further, the amino acid can be used not only in the free form but also as salts with organic acid or inorganic ion.

There is no particular restriction for the purity of the amino acid but it is preferably 90% or higher, more preferably, 95% or higher and, further preferably, 98.5% or more.

In the method of the present invention, the amino acid is dissolved in an aqueous medium for use. The concentration of the amino acid in the aqueous medium is 0.01% (w/v) to 50% (w/v), preferably, 0.1% (w/v) to 20% (w/v).

Any aqueous medium may be used so long as it gives no undesired effects on hatching eggs upon administration. For example, physiological saline, purified water such as distilled water, or buffer solution is used. The pH of the buffer solution is 6 to 8, preferably, 6.8 to 7.8 and, particularly preferably, 7.4. As the buffer solution, a phosphoric acid buffer solution, tris-hydrochloric acid buffer solution and HEPES hydrochloric acid buffer solution are used.

The aqueous medium may contain antioxidant, solubilizing aid, isotonic agent, surfactant and corrosion inhibitor or the like, so long as it give no undesired effects on hatching eggs upon administration.

As the antioxidant, ascorbic acid, vitamin E and L-cysteine are mentioned. As the solubilizing aid, polyethylene glycol is mentioned. As the isotonic agent, glycerine, glucose and sodium chloride are mentioned. As the surfactant, HCO-60(manufactured by Nikko Chemical Co.) is mentioned. As the corrosion inhibitor, phenol, sodium edetate, benzalconium chloride, citric acid, chloro-cresol, chloro-butanol, sodium salicylate, ethyl paraoxy benzoate and butyl paraoxy benzoate are mentioned.

The amino acid solution prepared as described above is usually used after sterilization for preventing infections of miscellaneous bacteria. As the method for sterilization, any ordinary sterilizing methods, such as autoclave digestion, filtration with filters or the like are used.

The amino acid solution may be prepared, for example, with reference to "Pharmacy", second edition, edited by Akinobu Otsuka, pp 141 - 161, published from Nankodo in 1995.

The amino acid may be used together with a vaccine. For use in the present invention, the vaccine is dissolved together with the amino acid in an aqueous medium. Preferably, the amino acid and the vaccine are mixed in an aseptic state.

As the vaccines, any vaccines conventionally used as the vaccines for hatching eggs may be used, and preferably, commercially available animal vaccines (vaccines contained in the Manual for Animal Pharmaceutics and Goods, 1998, 355 - 456 p, edited by the Foundation of Animal Pharmaceutical Associates of Japan) are used. For example, vaccine for Newcastle disease, infectious bronchitis vaccine, fowlpox vaccine, infectious coryza vaccine, infectious larynagotrachetis vaccine, mycoplasma gallicepticum vaccine, infectious fabricius bursa vaccine, aivian encephalomyetitis vaccine, laying lowering syndrome vaccine, mallec disease vaccine, infectious salmonella enteritides vaccine and avian pneumo virus vaccine are used alone or in combination.

The titer of the vaccine in the composition may be any titer so long as the vaccine acts effectively at the titer. Since the effective titer varies depending on vaccines, it is determined by reference, for example, to "vaccines contained in the Manual for Animal Pharmaceutics and Goods, 1998, 355 - 456 p, edited by the Foundation of Animal Pharmaceutical Associates of Japan".

When the amino acid and the vaccine are mixed, 0.1 to 100 parts of vaccine are mixed and stirred based on one part of amino acid.

When the amino acid and the vaccine are mixed, the concentration of the vaccine decreases, and in that case, the amount of the mixed solution to be administered may be increased. For instance, when the amino acid and the vaccine are mixed each in an equal amount, the amount of the mixed solution is increased twice.

The composition for improving the hatchability of eggs of the present invention (hereinafter simply referred to as a composition of the present invention) may be in the form of a solution containing the amino acid obtained as described above, or in the form of a freeze-dried product obtained by freeze drying the solution containing the amino acid by a conventional method.

The method of improving the hatchability of hatching eggs using the composition of the present invention is described below.

### 2. Method of Improving Hatchability of Eggs

After preparing the composition of the present invention obtained as described above, the composition of the present invention is administered into hatching eggs.

In a case where the composition of the present invention is solid such as a freeze-dried product, the solid can be used after it is dissolved in sterilized water. The concentration after dissolution is preferably 0.01% (w/v) to 50% (w/v) and, more preferably, 0.1% (w/v) to 20% (w/v). As the hatching eggs which can be improved for the hatchability in the present invention, hatching eggs of poultry and water fowls such as chickens, turkeys and ducks are mentioned.

There is no particular restriction for the timing of administrating the composition of the present invention into hatching eggs, and it is preferably at 10th to 20th days from laying.

The amount of the composition of the present invention to be administrated depends on the size of hatching eggs, and preferably, the amount is 0.05 to 0.5 ml per one hatching eggs.

The composition of the present invention is administered to amniotic fluid of the hatching egg. For preventing contamination with miscellaneous bacteria, preferably, a sterilized amino acid solution is administered by a sterilized injector (for example, manufactured by Beckman Dickinson Co.). Further, if necessary, administration is conducted while sterilizing the periphery of the administered area with 70% ethanol or tincture of iodine in order to prevent contamination with bacteria. After the administration, the administered area is sealed with paraffin or the like, if necessary.

Specifically, an Inovoject (trade name) administration system of Embrex Co. (1035, Swabia Cout Durham, NC27703, USA), which can aseptically treat 20,000 to 50,000 of eggs per hour, can be used preferably.

The amino acid-administrated hatching eggs can be incubated and hatched in the same manner as usual.

The hatchability of the hatching eggs can be improved by the method of the present invention.

A mode for carrying out the invention is described below, but the present invention is not limited thereto.

### Example 1: Preparation of amino acid solution-1 (Compositions 1 - 5)

Five test tubes each containing 10ml of physiological saline were prepared, and arginine was added to test tubes by 50 mg, 100 mg, 200 mg, 500 mg and 1 g, respectively, dissolved and then autoclaved for sterilization (120°C, 10 min).

As a result, L-arginine solutions at 0.5%, 1%, 2%, 5% and 10% (w/v) (as Compositions 1, 2, 3, 4 and 5, respectively) were obtained.

### Example 2: Preparation of amino acid solution-2 (Compositions 6 - 9)

Five test tubes each containing 10 ml of physiological saline were prepared, and L-arginine L-glutamete was added to the test tubes by 50 mg, 100mg, 200mg, 500 mg and 1 g, respectively, dissolved and autoclaved for sterilization (120°C, 10 min).

As a result, L-arginine L-glutamate solutions at 0.5%, 1%, 5% and 10% (w/v) (as Compositions 6, 7, 8 and 9, respectively) were obtained.

### Example 3: Preparation of amino acid solution-3 (Compositions 10 - 13)

Five test tubes each containing 10 ml of physiological saline were prepared and L-arginine acetate was added to the test tubes by 50 mg, 100 mg, 200 mg, 500 mg and 1 g, respectively, dissolved and autoclaved for sterilization (120°C, 10 min).

As a result, L-arginine acetate solutions at 0.5%, 1%, 5% and 10% (w/v) (as Compositions 10, 11, 12 and 13, respectively) were obtained.

### Example 4: Preparation of amino acid solution-4 (Compositions 14 - 18)

Five test tubes each containing 10 ml of physiological saline were prepared, and L-citrulline was added to the test tubes by 50 mg, 100 mg, 200 mg, 500 mg and 1 g, respectively, dissolved and autoclaved for sterilization (120°C, 10 min).

As a result, L-citrulline solutions at 0.5%, 1%, 2%, 5% and 10% (w/v) (as Compositions 14, 15, 16, 17 and 18, respectively) were obtained.

### Example 5: Administration of amino acid solution to chicken eggs-1

Compositions obtained in Examples 1-4 were administered to groups of hatching eggs of broilers(Cobb X Cobb), each group consisting of 10 eggs, in an amount of 0.1 ml with a sterilized injector on 18th day from incubation. A group administered with physiological saline instead of the amino acid solution and a group administered with nothing were used as controls, and the hatchability of eggs was compared.

Hatchability is shown in Table 1.

**Table 1**

| Composition No. | Hatchability(%) |
|---|---|
| not injected | 60 |
| Physiological saline | 60 |
| 1 | 100 |
| 2 | 80 |
| 4 | 90 |
| 5 | 100 |
| 6 | 100 |
| 7 | 80 |
| 8 | 80 |
| 9 | 80 |
| 10 | 90 |
| 11 | 90 |
| 12 | 90 |
| 14 | 100 |
| 15 | 100 |
| 17 | 90 |
| 18 | 90 |

While the hatchability was 60% in both of the control groups, the hatchability in the groups administered with Compositions 1 to 18 were remarkably increased to 80 to 100%.

### Example 6: Administration of amino acid solution to chicken eggs-2

Compositions 1, 2, 3, 14, 15, 16 obtained in Examples 1 to 4 were administered to groups of hatching eggs of broilers (Ross X Arbor Acre), each group consisting of 144 eggs, at a dose of 0.1ml on 18th day from incubation. A group administered with physiological saline instead of the amino acid solution was used as control, and the hatchability and body weight of nestling at 0th days from the hatching were measured for each of the groups.

The results are shown in Table 2.

**Table 2**

| Composition No. | Hatchability(%) | Body weight (g) |
|---|---|---|
| Physiological saline | 71.6 | 37.9 |
| 1 | 80.6 | 37.9 |
| 2 | 76.4 | 38.1 |
| 3 | 84.8 | 38.7 |
| 14 | 81.3 | 38.4 |
| 15 | 86.2 | 38.4 |
| 16 | 86.3 | 37.9 |

The groups to which the amino acid solution was administered were improved both in the hatchability and in the body weight of the nestling compared with the control group.

### Example 7: Preparation of vaccine-containing amino acid solution

In the same manner as in Example 1, vaccines for Newcastle disease, infectious bronchitis vaccine, fowlpox vaccine, infectious coryza vaccine, infectious larynagotrachetis vaccine, mycoplasma gallicepticum vaccine, infectious fabricius bursa vaccine, aivian encephalomyetitis vaccine, laying lowering syndrome vaccine, mallec disease vaccine, infectious salmonella enteritides vaccine and avian pneumo virus vaccine each containing 0.1 to 20% (w/v) of arginine were prepared respectively.

The titer for each vaccine was determined by reference to the descriptions in the "vaccines contained in the Manual for Animal Pharmaceutics and Goods, 1998, 355 - 456 p, edited by the Foundation of Animal Pharmaceutical Associates of Japan".

In the same manner as in Example 4, vaccines for Newcastle disease, infectious bronchitis vaccine, fowlpox vaccine, infectious coryza vaccine, infectious larynagotrachetis vaccine, mycoplasma gallicepticum vaccine, infectious fabricius bursa vaccine, aivian encephalomyetitis vaccine, laying lowering syndrome vaccine, mallec disease vaccine, infectious salmonella enteritides vaccine and avian pneumo virus vaccine each containing 0.1 to 20% (w/v) of citrulline were prepared respectively.

## Claims

1. A method of improving the hatchability of eggs, which comprises administrating an amino acid solution to hatching eggs.

2. A method as defined in claim 1, wherein the amino acid is a basic amino acid.

3. A method as defined in claim 2, wherein the basic amino acid is arginine or citrulline.

4. A method as defined in claim 1 or 2, wherein the amino acid solution is administrated together with a vaccine.

5. A method as defined in any one of claims 1 to 4, wherein the concentration of the amino acid in the amino acid solution is 0.01% (w/v) to 50% (w/v).

6. A method as defined in any one of claims 1 to 5, wherein the amount of amino acid to be administrated is 0.01 mg to 1 g per one administration.

7. A composition for improving the hatchability of eggs which contains an amino acid as an effective ingredient.

8. A composition as defined in claim 7, wherein the amino acid is a basic amino acid.

9. A composition as defined in claim 7 or 8, wherein the basic amino acid is arginine or citrulline.

10. A composition as defined in any one of claims 7 to 9, wherein the composition contains a vaccine.

11. A composition as defined in any one of claims 7 to 10, wherein the concentration of the amino acid is 0.01% (w/v) to 50% (w/v).

12. A composition as defined in any one of claims 7 to 11, wherein the composition is in the form of an aqueous solution.

13. A composition as defined in any one of claims 7 to 11, wherein the composition is in the form of a freeze-dried product.

14. A composition for improving the hatchability of eggs as defined in claim 12, wherein the aqueous solution is obtained by dissolving the composition as determined in claim 13 in water.
